# EUROPEAN PATENT APPLICATION

(11) **EP 4 640 183 A1**
(43) Date of publication of application: **29.10.2025**
(21) Application number: 25171657.7
(22) Date of filing: 22.04.2025
(51) Int. Cl.: A61F 2/00

(54) **FABRIC-LIKE HERNIA PLUG**

(30) Priority: 26.04.2024 US 202463639127 P
(71) Applicant: Morning Lily Surgical Innovations Inc., Sherwood Park, Alberta T8B 1M4 (CA)
(72) Inventor: FAROOQ, Umar, Sherwood Park (CA); DAVIS, Michael Loren, Shorewood (US)
(74) Representative: Wilson Gunn

(57) **Abstract**

Disclosed examples generally relate to a fabric-like smart hernia plug (202) and method of manufacturing thereof. In some examples, the hernia plug comprises an expandable mesh portion (206) formed of a smart memory alloy (SMA), wherein the mesh portion is transformable between a compressed pre-activated state and an expanded post-activated state, the mesh portion comprising a plurality of open cells surrounded by struts formed of the SMA.

## Description

### FIELD

Disclosed examples generally relate to hernia repair devices and, in particular, to a fabric-like hernia plug device.

### BACKGROUND

Hernias are medical defects characterized by the protrusion of an internal organ, or other body part, through a muscle or tissue normally containing the organ or body part.

Hernias are classified into various types based on their location, including: (i) femoral hernias (i.e., hernias occurring at the top of the inner thighs); (ii) umbilical hernias (i.e., hernias occurring near the naval); (iii) hiatal hernia (i.e., hernias occurring at the chest, through the diaphragm); and (iv) inguinal hernias (i.e., hernias occurring in the groin region, and the area between the lower part of the abdomen and the thigh). Among these hernia classes, inguinal hernias are among the most common type and affects all genders.

### SUMMARY

In at least one broad aspect, there is provided a hernia plug comprising a mesh portion transformable between a compressed pre-activated state and an expanded post-activated state, and the mesh portion comprises a plurality of open cells surrounded by struts formed of a smart memory alloy (SMA).

In some examples, the SMA comprises a biocompatible material.

In some examples, the SMA is a nickel-titanium alloy (nitinol).

In some examples, the open cells in the mesh portion have a fully connected design.

In some examples, in the compressed state, the plug is configured as an elongate tubular member.

In some examples, in the compressed state, the plug is receivable into an elongate deployment device.

In some examples, in the expanded state, the mesh portion expands from a radial center area to a radial outer periphery, and the mesh portion has a larger radius in the expanded state compared to the compressed state.

In some examples, the open cells near the radial outer periphery have a surface area greater than the open cells near the radial center.

In some examples, the open cells at the radial outer periphery have terminal ends which comprise curved or straight edges.

In some examples, one or more terminal ends are coupled together with linkage portions.

In some examples, the hernia plug further comprises a cover material that covers over at least a portion of the outer radial periphery of the mesh portion.

In some examples, the cover material is formed of polytetrafluoroethylene (PTFE) or expanded PTFE (ePTFE).

In some examples, each open cell is expandable along at least two axis.

In some examples, one or more suture strings are coupled to the hernia plug.

In some examples, a central area of the mesh portion includes one or more fastening apertures, and the sutures are coupled to the fastening apertures.

In another broad aspect, there is provided a method of manufacturing the hernia plug comprising: cutting the smart memory alloy (SMA) to form the plurality of open cells surrounded by struts; and shaping the SMA between the compressed pre-activated state and the expanded post-activated state

In different embodiments, the present invention may comprise a hernia plug or method of manufacturing thereof, comprising any combination of elements or features described herein, or which specifically omits any particular feature or element described herein.

### BRIEF DESCRIPTION OF THE DRAWINGS

In the drawings, like elements may be assigned like reference numerals. The drawings are not necessarily to scale, with the emphasis instead placed upon the principles of the present disclosure. Additionally, each of the embodiments depicted are but one of a number of possible arrangements utilizing the fundamental concepts of the present disclosure.
FIG. 1A is a schematic illustration of an example inguinal hernia.
FIG. 1B is a schematic illustration of an example conventional two-dimensional (2D) mesh applied to a hernia defect site.
FIGs. 2A - 2C provide various illustrations of the disclosed hernia plug according to at least one example, including illustrating the hernia plug from a side view (FIG. 2A), top view (FIG. 2B) and bottom isometric view (FIG. 2C).
FIG. 2D is a top plan view of the disclosed hernia plug, according to some other examples.
FIGs. 3A - 3D show various other example configurations for the hernia plug, with different terminal end designs.
FIGs. 4A - 4B illustrate the disclosed hernia plugs in a deployed and post-activated state.
FIGs. 5A - 5C illustrate a side profile view of the disclosed hernia plug in various activation states, including a pre-activated state (FIG. 5A), a partially activated state (FIG. 5B) and a post-activated state (FIG. 5C).
FIG. 6A is a cross-sectional view of the disclosed hernia plug, along the section line 6A-6A' in FIG. 5A.
FIG. 6B is a cross-sectional view of disclosed hernia plug, along the section line 6B-6B of FIG. 5C.
FIG. 7A illustrates a hernia plug, in a compressed or pre-activated state, received inside of a deployment tool.
FIG. 7B illustrates various sizes of hernia plugs in an expanded or post-activated state.
FIG. 7C illustrates various sizes of hernia plugs received inside of different deployment tools.
FIG. 8 illustrates an example hernia plug, in an expanded or post-activated state, with a cover material applied over the hernia plug.
FIG. 9 is an example process for a method of using the disclosed hernia plug.
FIGs. 10A - 10G illustrate various stages of deploying the disclosed hernia plug, including making an initial incision (FIG. 10A), inflating an inflatable medium (FIG. 10B), injecting liquid between the abdominal wall and peritoneum layer (FIG. 10C), applying a heating element to the hernia (FIG. 10D), inserting the hernia plug in the compressed or pre-activated state (FIG. 10E), allowing the hernia plug to expand into the expanded or post-activated state (FIG. 10F) and securing the hernia plug (FIG. 10G).

### DETAILED DESCRIPTION OF THE EMBODIMENTS

Examples herein generally relate to a fabric-like hernia plug device.

### I. GENERAL OVERVIEW

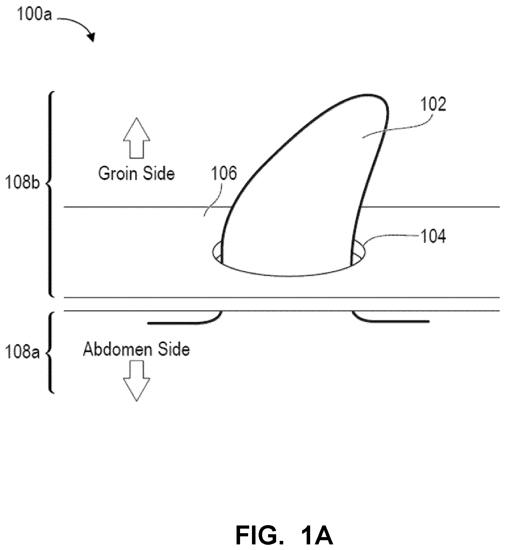

FIG. 1A shows a schematic illustration 100a of an example inguinal hernia. As shown, an internal organ 102 protrudes through the breach 104 in the abdominal wall 106. In this manner, the organ 102 protrudes from the lower abdomen side 108a and into the groin side 108b.

When a hernia defect is detected, surgical procedures are typically necessary to treat the hernia. While there is no definitive standard for these procedures, a routine procedure involves using a prosthetic mesh applied at the location of the hernia site.

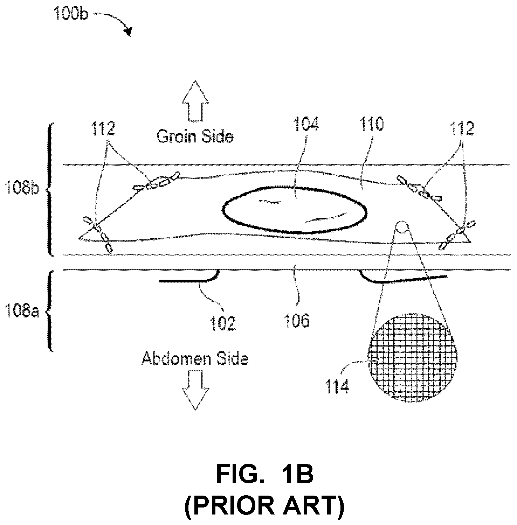

For example, FIG. 1B shows a schematic illustration 100b of a two-dimensional (2D) mesh 110 applied over the breach area 104. The mesh 110 is composed of a plurality of small pores 114. Mesh 110 may be secured in place by one or more fixing mechanisms 112 (i.e., sutures) applied by the surgeon. The mesh 110 functions to push the protruded organ 102 back into the abdomen side 108a to repair the hernia defect.

Use of conventional meshes are, however, associated with a number of drawbacks. For example, conventional meshes result in poor quality of tissue regrowth, and are associated with tearing and bleeding at the hernia site. The treatment of inguinal hernias using static synthetic meshes is also not entirely tension free, and is associated with tearing, bleeding, hematoma as well as nerve entrapment. These side-effects can cause severe discomfort and pain, instead of regenerating the weakened tissue.

Accordingly, there is a desire for a hernia plug that minimizes patient pain, reduces complications rates and simplifies procedure complexity.

### II. FABRIC-LIKE HERNIA PLUG

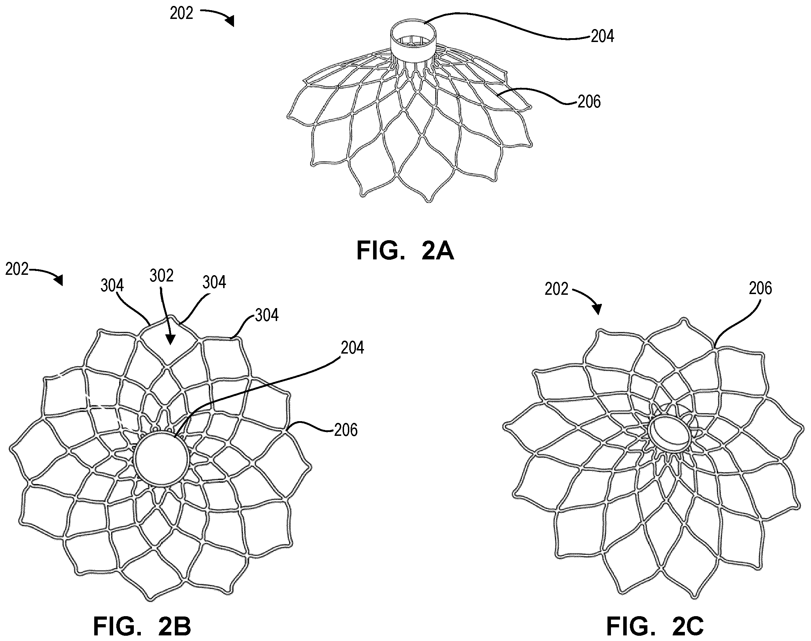

FIGs. 2A - 2D illustrate examples of a fabric-like hernia plug 202, in accordance with disclosed examples.

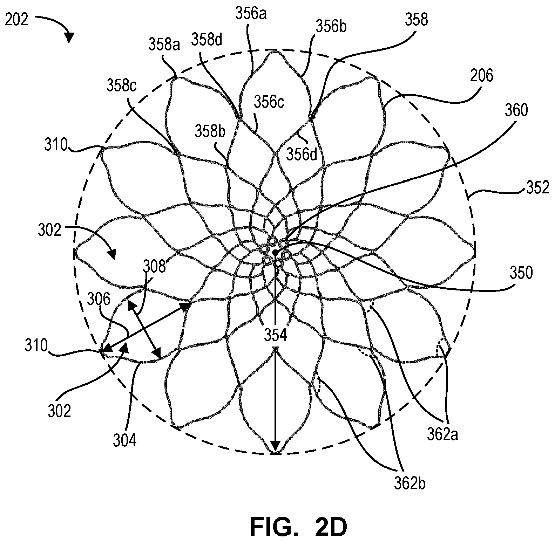

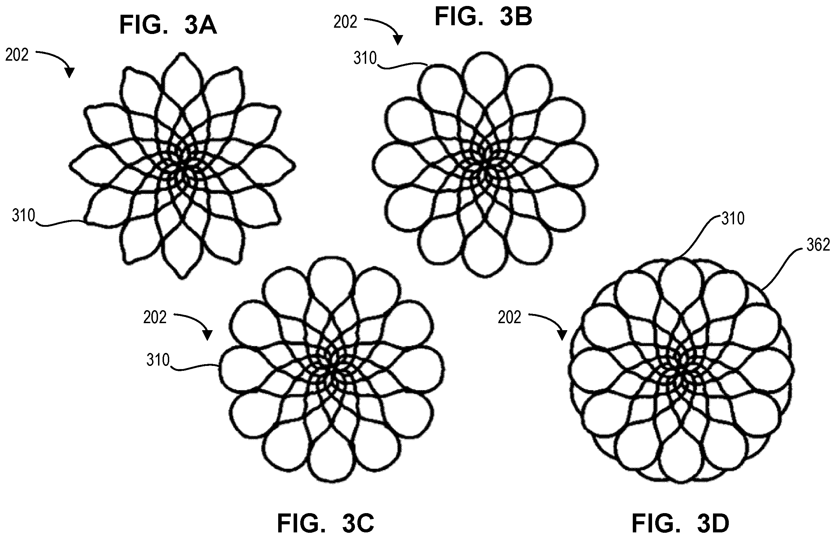

At a general level, hernia plug 202 includes two components: (i) a stem 204, and (ii) a mesh portion 206 (see FIGs. 2A - 2C). In some examples, the stem 204 is removed, and the hernia plug 202 includes only the mesh portion 206 (FIGs. 2D and 3).

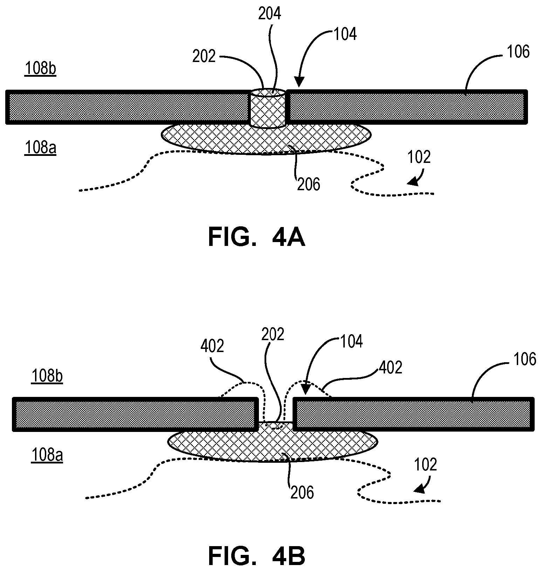

FIGs. 4A - 4B exemplify use of the hernia plug 202 where the plug includes the stem 204 (FIG. 4B) and excludes the stem 204 (FIG. 4B).

As shown in these figures, in use, the hernia plug 202 is deployed to the hernia breach site 104, and is positioned below the abdominal wall 106. In this position, the plug 202 pushes the hernia 102 downward and back into the abdominal side 108a. The plug 202 is effectively positionally secured between the hernia 102 and the abdominal wall 106. That is, the upward force of the hernia 102, and the limiting force of the abdominal wall 106, prevent the hernia plug 202 from being inadvertently displaced, e.g., moving.

In some examples, as shown in FIG. 4B, one or more securing mechanisms 402 (e.g., sutures) are applied to secure the hernia plug 202 to the abdominal wall 106, e.g., the groin-side of the abdominal wall. This can be performed to further secure the hernia plug 202 in position. In at least one example, the securing mechanisms 402 are used where the plug 202 does not include a stem 204.

In some cases, the securing mechanism 402 are sutures made wholly or partially of Kevlar^{™} material. This is owing to its strong material, lightweight, high temperature, and chemical and strain resistance, which are ideal for disclosed applications.

To this end, a unique feature of the hernia plug 202 is the material which forms the plug. Unlike conventional meshes 110 (FIG. 1B) which are formed of fabric, the disclosed hernia plug 202 is formed fully or partially of a shape memory alloy (SMA). In at least one example, the mesh 206 is formed of SMA.

While the hernia plug 202 may be formed of any desired SMA, in at least one example, the SMA comprises nickel titanium, also known in the art as "nitinol". More broadly, nitinol has a number of important appreciated advantages well-suited for the hernia plug application:

First, as nitinol is a type of "smart" memory alloy, it experiences shape memory effect (SME). As explained herein, SME is exploited to transform the shape of the hernia plug 202 between a compressed state (or a pre-activated state) and an expanded state (or a post-activated state). In the compressed state, the hernia plug 202 can be deployed through the narrow hernia breach site 104. In the expanded state, the hernia plug 202 expands into the configuration exemplified in FIGs. 2, 3, 4A and 4B to block the hernia breach 104, and secure the hernia 102 below the abdominal wall 106. Accordingly, in some examples, the mesh portion 206 is also referenced herein as an "expandable" mesh.

Second, despite nitinol's ability to transform between different shapes resulting from the shape memory effect (SME) - nitinol is also a rigid and resilient alloy material. Accordingly, in the expanded state (FIGs. 2, 3 4A and 4B), nitinol can ensure that the hernia 102 is securely pushed below the abdominal wall 106, while counteracting forces pushing the hernia 102 back through the breach site 104. For example, this includes counteracting forces applied by the human gut applying pressure pushing the hernia 102 back through breach site 104.

Third, nitinol is widely regarded as being a biocompatible material. Therefore, despite being a metal alloy, it still allows the hernia plug 202 to be deployed permanently (or temporarily) at a hernia defect site 104, with minimal deleterious effects to the patient's body.

In other examples, however, the hernia plug 202 may be formed of any other suitable SMA, including any biocompatible SMA. For example, these include copper-based alloys, iron-based alloys, Ni-free titanium alloys. In these cases, the hernia plug 202 is still able to benefit from the smart memory effect (SME) to allow it to transform between a compressed state (or a pre-activated state) and an expanded state (a post-activated state).

In examples provided herein, the SMA used to fabricate the hernia plug 202 (e.g., nitinol) is manufactured to provide a "fabric-like" feel to the plug. This allows the hernia plug 202 to adapt its shape to the surrounding organs when the hernia plug 202 is deployed (FIG. 4), thereby minimizing potential damage to these organs. In this manner, the hernia plug 202 may present both resilient mechanical properties (e.g., resulting from its metal alloy composition), as well as more flexible fabric-like features.

The disclosed fabric-like smart hernia plug 202 is now described in greater detail herein.

### III. SHAPE MEMORY ALLOY (SMA) FABRICATION

As noted above, the disclosed hernia plug 202 comprises (e.g., is composed of, fabricated from, or formed of) a shape memory alloy (SMA).

Shape memory alloys (SMAs) are a special branch of smart materials that display shape memory effect (SME). SME is the ability of such a material to recover from a temporarily assigned shape, to an original shape, in response to the particular stimuli. SMAs that activate (i.e., change shape) in response to thermal stimuli are known as "thermo-responsive" SMAs.

As known in the art, SMAs change between two phases: (i) a martensite crystal structure, and (ii) an austenite crystal structure.

The SMA takes on the austenite structure when the alloy is heated, starting from when the alloy is heated above the austenite start (Aₛ) temperature and completing the transformation when the temperature exceeds the austenite final (A_{f}) temperature.

In contrast, the SMA takes on the martensite crystal structure when the alloy is reverse cooled, with the transformation starting from when the alloy is cooled below the martensite start (Mₛ) temperature, and completing the transformation when the alloy is further cooled below the martensite final (M_{f}) temperature.

The SMA can also transition between the martensite and austenite phases in response to applied stress and strain.

More generally, when an SMA is in martensite phase, the metal is more easily deformed into any shape. In the austenite phase, the metal remembers its shape prior to deformation.

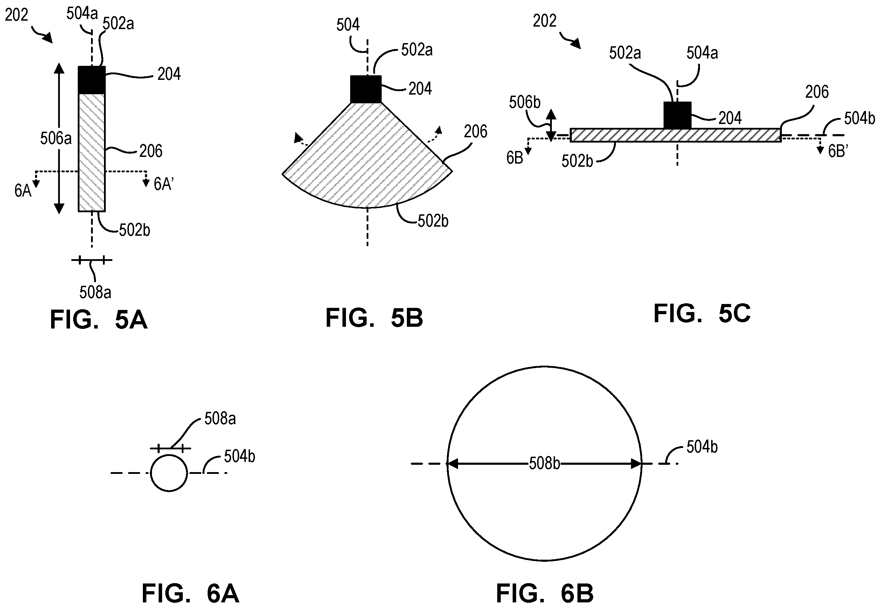

This smart memory effect (SME) functionality of SMAs allows the hernia plug 202 to be configured to transform between: (i) a "pre-activated" or compressed state (FIG. 5A), and (ii) a "post-activated" or expanded state (FIG. 5C).

In the "pre-activated" or compressed state (FIG. 5A), the hernia plug 202 has a generally narrower, elongated form (e.g., a tubular or cylindrical form) such that the mesh portion 208 wraps around a hollowed interior. In this elongated form, the mesh portion 208 generally extends along an extension axis 504a, as described herein.

In some examples, in the pre-activated state, the hernia plug 202 has a cylindrical form (e.g., tubular form) with a narrow diameter 508a (FIG. 6A). This allows the hernia plug 202 to be received inside of a cylindrical delivery tool (e.g., a tube).

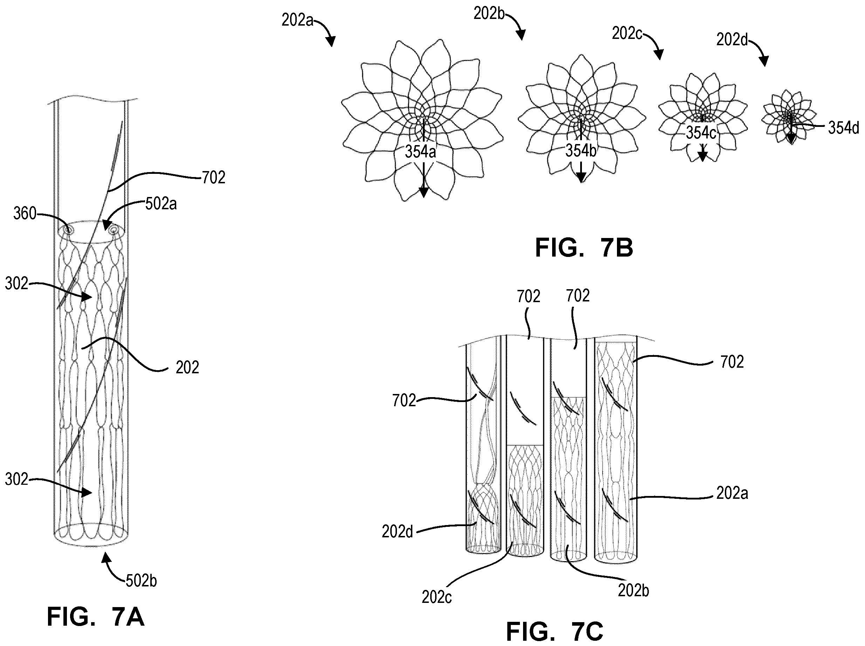

For example, in FIG. 7A, the compressed hernia plug 202 is received in a narrow cylindrical deployment tool or device 702. As explained below, this allows inserting the deployment tool 702 (carrying the compressed hernia plug 202), through the narrow hole defining the hernia breach 104, and inserting the hernia plug on the abdominal side 108a of the abdominal wall 106. In some examples, the hernia plug 202 is in a martensite phase when in the "deformed" pre-activated or compressed state.

In contrast, in the "post-activated" or expanded state, the hernia plug 202 transforms into an expanded configuration, which is exemplified in FIGs. 3 - 4 and 5C.

In the expanded state, the hernia plug 202 "fans out" to secure the hernia plug 202 below the abdominal wall 106, and pushes the hernia 102 below the abdominal-side 108a of the wall 106. In the post-activated state, the mesh portion 206 expands (e.g., fans out) radially outwardly. In FIG. 6B, it is observed the hernia plug 202 has an expanded diameter 508b relative to FIG. 6A, corresponding to the diameter of the mesh portion 206.

In some examples, the mesh 206 expands outwardly until it is generally flattened (FIG. 2D). For example, as shown in FIG. 5B, the mesh portion 206 can gradually expand such that it rotates approximately 90° relative to the extension axis 504a, and extends along an axis plane 504b generally orthogonal to the extension axis 504a. In some examples, in the post-activated state, the mesh portion 206 rotates between 0°< 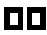 90°, and more preferably, 45° 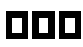 90°, relative to the extension axis 504a.

In the post-activated state, the hernia plug 202 may either be in a martensite or austenite phase.

As further shown in FIGs. 4A - 4B, in the post-activated and expanded state, the hernia plug 202 is prevented from slipping out of the hernia defect site 104. As noted previously, in the expanded state, the mesh portion 206 is compressed between the hernia 102 and the abdominal wall 106. This allow the hernia plug 202 to be at least partially secured in it is position.

Accordingly, the combination of using a smart memory alloy (SMA) enables inserting the hernia plug 202 through a narrow breach site 104 in a compressed state (FIG. 5A), and thereafter, allowing the hernia plug 202 to transform into its expanded state (FIG. 5C).

As provided herein, the hernia plug 202 may transform from the pre-activated compressed state to the post-activated expanded state through one or more of: (i) thermal activation; and (ii) applied force or stress.

With respect to thermal activation, the hernia plug 202 can transform into the expanded state owing to the temperature difference inside the human body. For example, the composition of the SMA used in the hernia plug 202 can be adjusted such that the SMA is thermally-activated at or near body temperature (e.g., 37°C).

In this manner, when the hernia plug 202 is deployed to the defect site 104, via a deployment tool, the body's temperature automatically transforms the hernia plug 202 from the compressed state into the expanded state (or at least, part way to an expanded state).

For example, the composition of the SMA material can be adjusted (using techniques well known in the art) such that, at body temperature, the hernia plug 202 transforms from the martensite phase to the austenite phase. If the hernia plug 202 is formed of nitinol, then as known in the art, the concentration (e.g., proportion) of nickel can be adjusted to vary the activation temperature (e.g., through trial and error, or through predefined concentration formulas).

In other examples, applied force or stress is used to transform the hernia plug 202 from the compressed to the expanded state. For example, as explained herein, sutures 402 can be used to apply force to expand the plug's mesh portion 202 into the expanded state. In some examples, if the thermo-activation was not sufficient to expand the mesh portion 206 into the fully expanded state, then further force or stress is applied (e.g., via sutures) to further expand the hernia plug 202 into the expanded state.

### IV. GEOMETRIC CONFIGURATION OF HERNIA PLUG

The following is a discussion of an exemplary geometric configuration for the hernia plug 202.

FIGs. 5A - 5C exemplify a side profile view of the hernia plug 202, and illustrating the hernia plug 202 in a compressed state (FIG. 5A), partially expanded state (FIG. 5B) and a fully expanded state (FIG. 5C).

At a general level, the smart hernia plug 202 extends between a first end 502a and a second end 502b. The ends 502a, 502b may be axially opposed along a longitudinal extension axis 504a.

A length dimension 506 is defined between the first and second ends 502a, 502b, along extension axis 504a. The length dimension 506a, in the compressed state (FIG. 5A) may be longer than the length dimension 506b in the expanded state (FIG. 5C).

In the illustrated example, the hernia plug 202 has a two-part composition: (i) a stem portion 204; and (ii) the mesh portion 206. In other examples, the stem 204 is omitted, and only the mesh portion 206 is included.

As exemplified in FIG. 2D, in the post-activated and expanded shape, the mesh portion 206 generally expands between a radial center area 350 and a radially outer area 352, along a radial dimension 354. In examples where a stem portion 204 is included, the stem 204 may be coupled (e.g., integrally coupled) to the radially center area 350.

As exemplified, in the expanded state, the mesh portion 206 can expand to have any desired shape, including a circular configuration or any other configuration that generally expands radially in all directions from a center area 350 (e.g., a daisy-shaped configuration). An advantage of using a radially expanding configuration is that the mesh portion 206 can more sturdily secure the hernia plug 202 in position from all sides.

In other examples, the mesh portion 206 can have any other shape in the expanded state, including shapes which only expand in certain radial directions (e.g., a semi-circular shape, or one or more radially expanding lobes).

To this end, the mesh portion 206 can also have any size dimension in the expanded state. For example, FIG. 7B exemplifies hernia plugs 202a - 202d with mesh portions 206 have various radial size dimensions 354a - 354d. In some examples, larger mesh portions 206 are more suited for application with larger hernia defects.

As shown in FIG. 7C, different sizes of hernia plugs 202, in the compressed state, are also receivable in different sizes of deployment tools 702. More generally, larger mesh portions 206 may require a deployment tool that accommodates a larger extension dimension 506a (FIG. 5A) and radial dimension 508a (FIG. 6A) in the compressed state.

Mesh portion 206 may also have one of a number of design patterns. In the illustrated example, as best shown in FIG. 2D, the mesh portion 206 is designed with a plurality of cut-out cells 302 (also referred to herein as "open cells" or "void cells"). Each open cell 302 may be fully bounded by corresponding strut members 304 (e.g., formed of a smart memory alloy (SMA)).

At least one appreciated advantage of using an open cell design is that it permits ingrowth of tissue within and between the open cells 302 when the hernia plug 202 is deployed at the hernia site, and in the expanded state. This, in turn, allows the mesh portion 206 to embed more securely at the hernia site 104, and for longer time durations. Another appreciated advantage of using an open cell design, which is explained below, is that a fabric can be weaved within the open cells 302 (e.g., around the strut members 304) to help better secure the hernia plug 202 inside the body (e.g., via suturing).

As exemplified, the mesh portions 206 can be configured with a fully connected design (e.g., a spider web design). As used herein, a *"fully connected"* design means that each open cell 302 shares a common strut 304 boundary with at least one other open cell 302, and more preferably, at least two other open cells 302. It may also be expressed as meaning a structural configuration in which each node 358 or connection point of the mesh is directly connected to every other node or element. This design ensures continuous interconnection across the mesh structure. This design may also be referenced as a tessellation mesh, such as a diamond tessellation mesh or rhombic tessellation mesh.

In some examples, in a fully connected design, the open cells 302 at the outer radial periphery 352 share a common strut boundary 304 with at least two other open cells 302. Further, open cells 302 which are located inwardly from the outer radial periphery 352 can share common strut boundaries 304 with at least four other surrounding open cells 304.

It is appreciated that using a fully connected design in conjunction with open cells 302 provides a resilient "fabric-like" feel to the hernia plug 202. The fabric-like feel primarily results from using thin struts 304 extending between the open cells 302, which provide the plug 202 with more flexibility and malleability. This flexibility allows the hernia plug 202 to flexibly deform and better accommodate the shape of the surrounding organs in the expanded state, without otherwise puncturing the organs.

In some examples, use of a fully connected design with struts 304 made of a smart memory alloy (SMA) provides a resilient sturdy structure to the hernia plug 202 (e.g., owing to the alloy fabrication), that is balanced with fabric-like flexibility.

In at least one example, the hernia plug's mesh portion 206 has a thickness of less than about 1 inch (± 1 inch), and more preferably, around about 0.01 inches (e.g., 0.012 inches). The smaller thickness enhances the fabric-like flexibility of the mesh portion 206.

In some examples, the length of each strut 304 - surrounding each open cell 302 - increases at greater radial distances 354 from the center point 350 (FIG. 2D), e.g., a linear increase.

For example, in FIG. 2D, the open cells 302 radially closer to the outer radial periphery 352 are larger, and are surrounded by a longer strut 304, than open cells 302 radially closer to the center area 350.

In at least one example, the bounded area - defined between struts 304 around a cell 302 - defines the surface area of that cell (e.g., in² or cm², depending on the measurement system used). Accordingly, open cells 302 radially closer to the outer periphery 352 have a larger area than those closer to the center 352. In some cases, cells 302 disposed at the same radial distance from the center 350 have the same area (and surrounding strut length). Accordingly, cells 302 have greater surface area at further radial distances. In some examples, the strut length is defined as the length of strut extending between two adjacent connection points 356.

An appreciated advantage of this design is that it allows the mesh portion 206 to expand from the contracted elongated shape (FIG. 5A), to an expanded flat shape (FIG. 2D).

For example, as shown in FIGs. 5A - 5C, in the process of expanding, the mesh portion 206 rotates by approximately 90° (e.g., relative to the extension axis 504a) as it expands from the compressed elongated cylindrical shape (FIG. 5A), to the expanded daisy-shape (FIG. 5C). In order to enable the mesh 206 to expand in this manner into a flat design (FIG. 5C), the radially outward open cells 302 will have a longer strut 304 boundary to accommodate the larger fan-out at the radial outer periphery 352. In this manner, mesh portion 206 is designed to provide a stable expansion and compression. As well, the longer struts at the radial periphery 352 allows the mesh portion 206 to expand fully to adopt a flat shape, which enables the hernia plug 202 to be more secure when deployed at the hernia site 104. This is also shown in FIG. 7A, whereby the cells 302 near the second end 502b in the compressed state have a longer axial length than cells 302 near the first end 502a.

In some examples, the larger open cells 302 at the periphery also add to the overall flexibility of the design. This is because the struts 304 decrease in tensile strength at the periphery 352, as compared to the center point 350, because of the longer struts 304.

To this end, the open cells 302 can have any desired shape. In the illustrated example, the open cells 302 have at least three strut borders 356a - 356d defined between at least three corners or connection nodes 358a - 358d (FIG. 2D). This forms a diamond mesh or rhombic mesh.

The borders may be separated, for instance, by angles of between 45° to 300°. In some examples, the angles 362a between the strut boundaries 356 along the radial axis (FIG. 2D) are separated by approximately 40° to 80°, and in some examples, 60° in the expanded state. Further, the angles 362b between the strut boundaries 356 orthogonal to the radial axis (FIG. 2D) are separated by approximately 100° to 140°, and in some examples, 120° in the expanded state. This defines, for example, a generally diamond-like, rhomboid, polygonal, lanceolate polygon, spade-like or mushroom-like shape for each open cell 302.

The use of open cells 302 having at least three strut borders and/or corners better enables the open cells 302 to expand in all radial directions when the mesh portion 206 is expanding.

For instance, as shown in FIG. 2D, by using four strut borders 304, the open cell 302 can have a radial dimension 306 (e.g., extending along a radial direction 354), and a width dimension 308 (e.g., extending along an axis orthogonal to the radial direction 354). The radial and width dimensions are able to vary to allow for expansions and contractions in multiple directions, and along at least two axis (e.g., 306 and 308). For example, during expansion, the radial dimension 306 decreases, while the width dimension 308 increases, and vice-versa during compression. As exemplified, the use of open cells 302 having the aforesaid design also provides the mesh portion 206 with a daisy-shaped configuration in the expanded state.

In other examples, the open cells 302 can have any desired number of borders 356 or connection nodes 358

In at least one example, the terminal ends 310 (or terminal corners) of each open cell 302 along the outer periphery 352 has a curved end (e.g., a key-hole type curve), e.g., with respect to the end 310 directed radially away towards the outer periphery 352.

The advantage of using a curved terminal end is that the protruding corner is less likely to puncture through surrounding tissue, which is a significant consideration in a hernia plug application. This ability of the hernia plug 202 to minimally damage the surrounding tissue is also referred to as a "reduced mechanical toxicity" feature of the hernia plug 202 (or otherwise, the plug is said to be "atraumatic").

FIGs. 3A - 3D show various other examples for the terminal ends 310. This includes a key-hole protruding curve (FIG. 3A), a full curve (FIG. 3B), or a flat end (FIG. 3C).

In some examples, as shown in FIG. 3D, there may be one or more linkage portions 362 that couple adjacent terminal ends 310 (e.g., formed of the same material). The advantage of this is to further reduce the mechanical toxicity of the plug by closing of the gaps between terminal ends.

In some examples, the central area 350 may include one or more fastening apertures 360 (FIG. 2D). These apertures 360 may receive a fixing mechanism, such as sutures, which are used for securing the device within the body.

### V. COVER MATERIAL

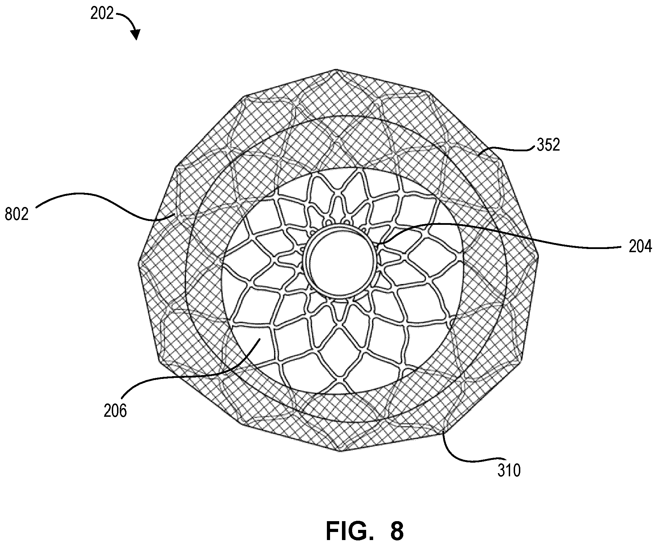

As exemplified in FIG. 8, a cover material 802 may be applied over the hernia plug 202, e.g., applied over and/or around the mesh portion 206. In some examples, the cover material 802 can be applied over and around the outer periphery 352 of the plug's mesh portion 206 (e.g., on one or both sides). The cover material 802 may allow for covering over the terminal ends 310 of the outer periphery open cells 302. In this manner, the cover material 802 minimizes the likelihood that the terminal ends 310 puncture the internal organs.

In at least one example, the cover material 802 is made of (e.g., composed of) biocompatible material. For example, this may include polytetrafluoroethylene (PTFE) (also known as Teflon^{™}). The cover material 802 can be made completely or at least partially of PTFE (e.g., a PFTE composite material). It is appreciated that PTFE stimulates growth of biological tissue. Accordingly, using the PTFE cover, the tissue is more likely to grow through and within the open cells 302, thereby more securely integrating the hernia plug 202 more permanently within the internal organ.

In some examples, the PTFE comprises expanded PTFE (ePTFE). The use of ePTFE is more microporous and permeable, which allows the hernia plug 202 to integrate better into the surrounding tissue. Further, ePTFE is more flexible, thereby allowing the hernia plug 202 to maintain a "fabric-like" feel.

In some examples, the cover material 802 is formed of polypropylene.

### VI. METHOD OF USING HERNIA PLUG

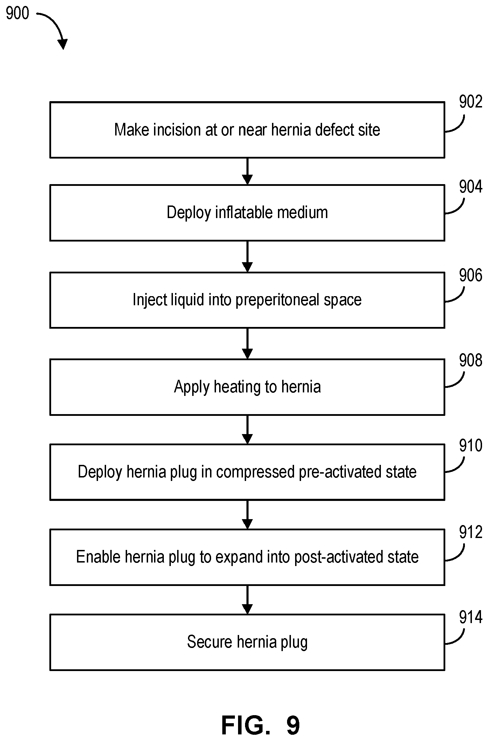

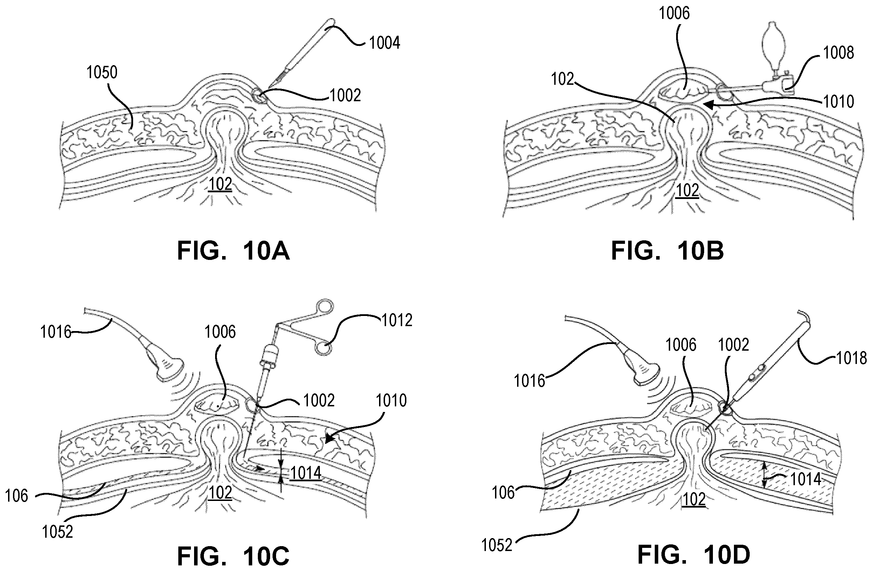

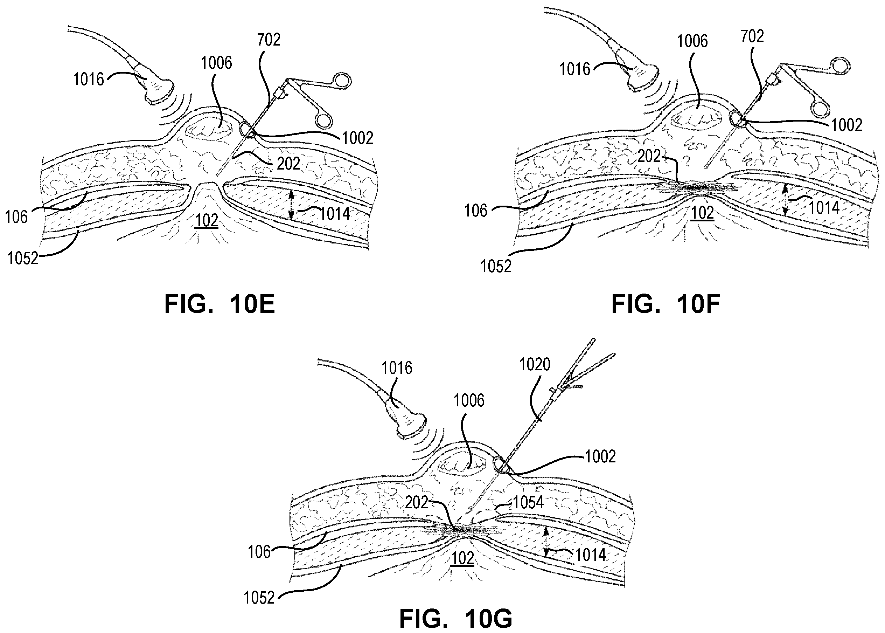

FIG. 9 shows an example method 900 for using and/or deploying the hernia plug 202 at a hernia defect site 104. Reference is also made concurrently to FIG. 10, which provides an illustration of the process of using the hernia plug in accordance with FIG. 9.

At 902, an incision is made at or near the hernia defect site. For example, this is shown as the incision 1002 beside the hernia 102 (e.g., the inguinal hernia) (FIG. 10A). The incision can be made, for example, using a scalpel, pair of scissors, or any other surgical tool known in the art. The surgical incision 1002 is a small incision, and in some examples, no wider than a 5 mm hole. The incision 1002 can extend through the layer of human fat 1050 and reaching to the hernia 102.

At 904, as shown in FIG. 10B, a conveyance device 1008 (e.g., a balloon catheter) is inserted through the incision 1002 and is used for deploying an inflatable medium 1006 above the hernia 102. For example, the inflatable medium 1006 comprises an inflatable balloon.

Once the conveyance device 1008 is inserted inside the incision 1002, the inflatable medium 1006 can be inflated with any fluid (e.g., liquid or gas) as known in the art. In some examples, the inflatable medium 1006 is inflated with carbon dioxide or oxygen.

The effect of inflating the medium 1006 is to provide an interspace 1010 (e.g., between the hernia 102 and other top body layers) to insert other tools into the hernia defect site. Once the medium 1002 is inflated, the conveyance tool 1008 can be removed.

In other examples, any other separation technique can be used for form the upper interspace 1010.

At 906, a liquid conveyance tool 1012 (e.g., a syringe) is inserted through the incision 1002, and is used to inject liquid (e.g., water and/or saline) or other medium between the abdominal layer 106 and the peritoneum layer 1052, e.g.. in the preperitoneal space 1014. This has the effect of expanding and separating the abdominal layer 106 from the lower peritoneum layer 1052.

More particularly, the peritoneum layer 1052 is mostly closely in contact with the rest of the subject's organs (e.g., gut). These organs often apply an outward force to push the hernia 102 out of the abdominal wall 106. Accordingly, to counteract this force, the liquid forces the abdominal wall 106 away from the remaining gut.

In some examples, a scanning unit 1016 is used to locate the peritoneum layer 1052, in order to correctly position the syringe 1012. Scanning unit 1016 can be, for example, an ultrasound device as known in the art (e.g., using a probe and computer display screen).

At 908, in some examples, a heating device 1018 is inserted through the incision 1002 (FIG. 10D). The heating device 1018 can include a heating rod or the like, and is applied to the portion of the hernia 102 above the abdominal wall 106. The result is that the top portion of the hernia 102 is cut away, and the hernia 102 is reduced in overall size. In some examples, only the portion of the hernia 102 below the abdominal wall 106 remains after the heating is applied. This provides sufficient room to allow deployment of the hernia plug 202 through the hernia defect site 104.

At 910, a deployment device 702 is then used to deploy the hernia plug 202 at the hernia defect site, and underneath the abdominal wall 106 (FIG. 10E). In some examples, this is guided by the scanning unit 1016. At this stage, the hernia plug 202 is in a compressed-preactivated state while it is disposed inside of the deployment device 702. Deployment device 702 can be an elongated tube or the like.

At 912, the deployed hernia plug 202 expands into the expanded post-activated state (FIG. 10F), in which the mesh portion 206 expands to push the hernia 102 under the abdominal wall 106. In some examples, the mesh 206 is dimensioned to be wider than the hernia defect site 104 when expanded.

As explained, the hernia plug 202 can transform into the expanded state at 912 in several ways. In some examples, the smart memory alloy (SMA) which forms the hernia plug 202 is thermo-activated at the body's temperature. Accordingly, once the hernia plug 202 is pushed out of the conveyance tool 1008, the body's temperature may cause the hernia plug 202 to transform into the expanded state. In other examples, as explained in the next step, force can be applied to further expand the mesh portion 206, e.g., if the thermo-activation did not sufficiently transform the mesh portion 206 into the expanded state.

At 914, in some examples one or more secure mechanisms 1054 (e.g., sutures) are used to secure the hernia plug 202 to the abdominal wall 106. For example, as shown in FIGs. 7A and 7C, the strings can be looped through some of the open cells 302 (or the fastening apertures 360 in FIG. 2D), and coupled to the hernia plug 202 while it is in conveyance tool 1008. A suturing tool 1020 can be inserted through the incision 1002 to facilitate adjusting the suture strings. In some examples, this suturing is performed to: (i) secure the hernia plug 202 inside the body; (ii) force the hernia plug 202 to further expand into its fully post-activated state if not already expanded due to the thermal-activation. In some examples, the sutures are made of Kevlar^{™} material.

In other examples, rather than suturing, the hernia plug is secured using any other suitable mechanism. For example, a glue may be applied, such as by using an extended glue gun that is inserted through the incision 1002. The glue is applied, for instance, between the abdominal wall 106 and the hernia plug 202.

In some examples, the hernia plug 202 is deployed under the peritoneum layer and not simply only in the preperitoneal space.

Here, it is appreciated that the entire deployment of the hernia plug 202 is performed through the small incision (e.g., a 5 mm hole), which is large enough to accommodate the insertion of the various conveyance and deployment devices. This, in turn, allows the hernia plug 202 to be deployed in a minimally intrusive manner, which does not require using a large incision cut to accommodate the entirety of the hernia plug (e.g., a 304 mm hole), or using multiple incisions.

It is also appreciated that some acts of method 900 can be performed in a different order. Further, it is possible that the hernia plug 202 is deployable without performing each of the method acts 902 - 914. For instance, in some cases, it is possible to deploy the plug 202 by performing only acts 902 and 910 - 912, with no further acts. In other cases, some (but not all) of acts 904 - 908, and 914 may be also performed to ensure more effective deployment.

### VII. METHOD OF MANUFACTURING HERNIA PLUG

In at least one example, the process of manufacturing the hernia plug 202 involves providing a shape memory alloy (SMA) in its expanded state (e.g., as a flattened material). The open cells and the outer design are then cutout, using a laser or another suitable cutting tool. The plug is then shaped into a compressed state, either through heat or stress application.

Alternatively, the SMA is initially provided in its compressed state and being in a tubular form. It is then expanded into an uncompressed state, either through heat or stress, which allows for the cutting of the open cells and defining the outer periphery design. It is also possible to cut the open cells while the SMA is still in its compressed state.

In some examples, the SMA is also cut to form the fastening apertures 360 (FIG. 2D).

In at least one example, prior to cutting or shaping - the SMA is initially produced to have a transition temperature that matches the body temperature (e.g., around 37°C). This can be achieved by adjusting the concentration of nickel to the right amount, if a nitinol material is used, as previously explained. In other examples, the SMA material is produced to have any other desired transition temperature.

### VIII. INTERPRETATION

Aspects of the present invention may be described with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

The flowchart and block diagrams in the Figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The corresponding structures, materials, acts, and equivalents of all means or steps plus function elements in the claims appended to this specification are intended to include any structure, material, or act for performing the function in combination with other claimed elements as specifically claimed.

References in the specification to "one embodiment", "an embodiment", " "embodiments," "the embodiment," "the embodiments," "one or more embodiments," "some embodiments," and "one embodiment" etc., indicate that the embodiment described may include a particular aspect, feature, structure, or characteristic, but not every embodiment necessarily includes that aspect, feature, structure, or characteristic. Moreover, such phrases may, but do not necessarily, refer to the same embodiment referred to in other portions of the specification. Further, when a particular aspect, feature, structure, or characteristic is described in connection with an embodiment, it is within the knowledge of one skilled in the art to affect or connect such module, aspect, feature, structure, or characteristic with other embodiments, whether or not explicitly described. In other words, any module, element or feature may be combined with any other element or feature in different embodiments, unless there is an obvious or inherent incompatibility, or it is specifically excluded.

It is further noted that the claims may be drafted to exclude any optional element. As such, this statement is intended to serve as antecedent basis for the use of exclusive terminology, such as "solely," "only," and the like, in connection with the recitation of claim elements or use of a "negative" limitation. The terms "preferably," "preferred," "prefer," "optionally," "may," and similar terms are used to indicate that an item, condition or step being referred to is an optional (not required) feature of the invention.

The singular forms "a," "an," and "the" include the plural reference unless the context clearly dictates otherwise. The term "and/or" means any one of the items, any combination of the items, or all of the items with which this term is associated. The phrase "one or more" is readily understood by one of skill in the art, particularly when read in context of its usage.

Some elements herein may be identified by a part number, which is composed of a base number followed by an alphabetical or subscript-numerical suffix (e.g. 112a, or 112₁). Multiple elements herein may be identified by part numbers that share a base number in common and that differ by their suffixes (e.g. 112₁, 112₂, and 112₃). All elements with a common base number may be referred to collectively or generically using the base number without a suffix (e.g. 112).

The term "about" can refer to a variation of ± 5%, ± 10%, ± 20%, or ± 25% of the value specified. For example, "about 50" percent can in some embodiments carry a variation from 45 to 55 percent. For integer ranges, the term "about" can include one or two integers greater than and/or less than a recited integer at each end of the range. Unless indicated otherwise herein, the term "about" is intended to include values and ranges proximate to the recited range that are equivalent in terms of the functionality of the composition, or the embodiment.

The terms "including," "comprising" and variations thereof mean "including but not limited to," unless expressly specified otherwise. A listing of items does not imply that any or all of the items are mutually exclusive, unless expressly specified otherwise. The terms "a," "an" and "the" mean "one or more," unless expressly specified otherwise.

As will be understood by one skilled in the art, for any and all purposes, particularly in terms of providing a written description, all ranges recited herein also encompass any and all possible sub-ranges and combinations of sub-ranges thereof, as well as the individual values making up the range, particularly integer values. A recited range includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to", "at least", "greater than", "less than", "more than", "or more", and the like, include the number recited and such terms refer to ranges that can be subsequently broken down into sub-ranges as discussed above. In the same manner, all ratios recited herein also include all sub-ratios falling within the broader ratio.

## Claims

1. A hernia plug comprising a mesh portion transformable between a compressed pre-activated state and an expanded post-activated state, and the mesh portion comprises a plurality of open cells surrounded by struts formed of a smart memory alloy (SMA).

2. The hernia plug of claim 1, wherein the SMA comprises a biocompatible material.

3. The hernia plug of any one of claims 1 or 2, wherein the SMA is a nickel-titanium alloy (nitinol).

4. The hernia plug of any one of claims 1 to 3, wherein the open cells in the mesh portion have a fully connected design.

5. The hernia plug of any one of claims 1 to 4, wherein in the compressed state, the plug is configured as an elongate tubular member.

6. The hernia plug of claim 5, wherein in the compressed state, the plug is receivable into an elongate deployment device.

7. The hernia plug of any one of claims 1 to 6, wherein in the expanded state, the mesh portion expands from a radial center area to a radial outer periphery, and the mesh portion has a larger radius in the expanded state compared to the compressed state.

8. The hernia plug of claim 7, wherein the open cells near the radial outer periphery have a surface area greater than the open cells near the radial center.

9. The hernia plug of any one of claims 7 or 8, wherein the open cells at the radial outer periphery have terminal ends which comprise curved or straight edges.

10. The hernia plug of claim 9, wherein one or more terminal ends are coupled together with linkage portions.

11. The hernia plug of any one of claims 1 to 10, further comprising a cover material that covers over at least a portion of the outer radial periphery of the mesh portion.

12. The hernia plug of claim 11, wherein the cover material is formed of polytetrafluoroethylene (PTFE) or expanded PTFE (ePTFE).

13. The hernia plug of any one of claims 1 to 12, wherein each open cell is expandable along at least two axis.

14. The hernia plug of claims 1 to 13, wherein one or more suture strings are coupled to the hernia plug.

15. A method of manufacturing the hernia plug of any one of claims 1 to 14 comprising:
- cutting the smart memory alloy (SMA) to form the plurality of open cells surrounded by struts; and
- shaping the SMA between the compressed pre-activated state and the expanded post-activated state.
